(19) Europäisches Patentamt
European Patent Office
Office européen des brevets

(11) **EP 4 012 042 A1**

(12) **EUROPEAN PATENT APPLICATION**

(43) Date of publication:
**15.06.2022 Bulletin 2022/24**

(21) Application number: **20213820.2**

(22) Date of filing: **14.12.2020**

(51) International Patent Classification (IPC):
*C12Q 1/24* (2006.01)    *A61B 10/00* (2006.01)
*G01N 33/497* (2006.01)    *G01N 33/569* (2006.01)

(52) Cooperative Patent Classification (CPC):
**G01N 33/569; C12Q 1/24; G01N 33/497;**
A61B 2010/0087

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Helmholtz-Zentrum für
Infektionsforschung GmbH
38124 Braunschweig (DE)**

(72) Inventors:
• **KRAUSE, Gérard**
  **38124 Braunschweig (DE)**
• **CASTELL, Stefanie**
  **38124 Braunschweig (DE)**
• **KERRINNES, Tobias**
  **38124 Braunschweig (DE)**
• **STRENGERT, Monika**
  **38124 Braunschweig (DE)**

(74) Representative: **Gramm, Lins & Partner
Patent- und Rechtsanwälte PartGmbB
Freundallee 13a
30173 Hannover (DE)**

(54) **ASSEMBLY FOR ENRICHMENT OF MICROORGANISMS PRESENT IN THE AEROSOL INCLUDING SMALL DROPLETS FROM RESPIRATORY AIR OF A SUBJECT AND METHOD FOR ENRICHMENT OF MICROORGANISMS**

(57)     The present invention relates in a first aspect to an assembly for enrichment of microorganisms present in the aerosol, containing/including droplets including droplets smaller than 5 $\mu$m from respiratory air of a subject, said assembly has at least one filter unit, at least one inlet and at least one outlet whereby at least one of the filter units comprise a first region containing beads, the liquids and solid particles in the aerosol, like droplets attach to the beads when respiratory air is introduced by the inlet and passed into the filter through the inlet on exhalation. In another aspect, a method for enrichment of microorganisms from respiratory air of a subject is provided comprising the steps of providing an assembly, having at least one filter unit, at least one inlet and at least one outlet collecting aerosol including droplets containing microorganisms from the respiratory air in the filter unit upon exhalation of the subject by passing the exhaled air, the aerosol, through the inlet to the filter unit whereby the filter unit having a region with beads for enrichment for the microorganisms present in the aerosol like in the droplets of the exhaled air. Further, a method for determining microorganisms in respiratory air of a subject is provided including collecting the microorganisms with an assembly having a filter unit as described and separating the microorganisms from the beads present in the filter unit allowing determination of microorganisms by appropriate methods. Finally, the use of the assembly according to the present invention for the enrichment of microorganisms present in aerosol including droplets from the respiratory air of a subject or for determining the presence of microorganisms in the respiratory air of a subject, in particular for determining the presence of viruses like enveloped viruses.

Figure 3

**Description**

The present invention relates in a first aspect to an assembly for enrichment of microorganisms present in the aerosol, like droplets including droplets smaller than 5 µm from respiratory air (expirate) of a subject, said assembly has at least one filter unit, at least one inlet and at least one outlet whereby at least one of the filter units comprise a first region containing beads, the liquids and solid particles in the aerosol, like droplets attach to the beads when respiratory air is introduced by the inlet and passed into the filter through the inlet on exhalation. In another aspect, a method for enrichment of microorganisms from respiratory air of a subject is provided comprising the steps of providing an assembly, having at least one filter unit, at least one inlet and at least one outlet collecting aerosol including droplets containing microorganisms from the respiratory air in the filter unit upon exhalation of the subject by passing the exhaled air, the aerosol, through the inlet to the filter unit whereby the filter unit having a region with beads for enrichment for the microorganisms present in the aerosol like in the droplets of the exhaled air. Further, a method for determining microorganisms in respiratory air of a subject is provided including collecting the microorganisms with an assembly having a filter unit as described and separating the microorganisms from the beads present in the filter unit allowing determination of microorganisms by appropriate methods. Finally, the use of the assembly according to the present invention for the enrichment of microorganisms present in aerosol including droplets from the respiratory air of a subject or for determining the presence of microorganisms in the respiratory air of a subject, in particular for determining the presence of viruses like enveloped viruses.

**Background of the invention**

Determining components or particles present in the respiratory exhalation air of a subject is of general interest. For example, determining the presence of pathogenic components or particles like microorganisms in the respiratory air is of particular interest in various diseases. Moreover, various biomarkers are known to be used in methods for diagnosing different medical conditions of patients whereby these biomarkers are traced in exhaled breath of subjects.

Exhaled air is an aerosol containing endogenously generated droplets. The droplets contain water and non-volatile material. Small droplets are often colloquially also called aerosol. Further, the droplets are often referred to as particles in general (B. Bake, et. al., Respiratory research 20, 8-8 (2019)).

Various diseases and conditions are associated with affecting lung or other compartments involved in breathing, invading for example mucosal tissue present in the airways of a subject. This includes the upper respiratory tract with the nasal cavity, the pharynx and the larynx and the lower respiratory tract with the lung, the trachea and the primary bronchi.

For example pneumonia or an inflammation of the lungs in general, is a leading cause of morbidity and mortality worldwide. Pneumonia can be caused by a variety of bacterial and viral pathogens, including *Streptococcus pneumoniae, Mycoplasma tuberculosis, Chlamydia pneumonia, Legionella,* but also viral pathogens like influenza viruses, respiratory syncytial virus, parainfluenza, adenovirus, rhinovirus, human bocavirus, *Mycoplasma pneumonia*, hantavirus and cytomegalovirus. Of course, corona viruses are within this group as well. The new corona virus, SARS-CoV-2 (severe acute respiratory syndrome coronavirus 2), is the strain of corona virus that causes corona virus disease 2019 (Covid-19) which is a respiratory illness belonging to the family of Coronaviridae, an enveloped positive-sense single stranded RNA virus. As noted, Covid-19 represents an infectious disease caused by the SARS-CoV-2. Further, MERS (Middle East Respiratory Syndrome) was described in 2012 first representing a viral respiratory infection as well.

To determine these components or particles in the respiratory tract and the respiratory air when breathing, coughing, talking or sneezing, either non-invasive or invasive techniques may be applied. Invasive techniques include laparoscopic alveolar lavage, lung puncture but also taking samples from the upper respiratory tract like with cotton swabs. However, for determining the components in the respiratory air which eventually include infectious pathogens, other sampling is desired. Namely, the components to be detected, determined and/or analysed may be present in the respiratory air in different forms. In the respiratory air aerosol with droplets is present, said aerosol with droplets contain components and particles like chemical compounds but also more complex particles including microorganisms. In general, aerosol contain liquid or solid particles suspended in air. They can be visible, like fog, bust most often are invisible, like dust or pollen. Nowadays, usually only small droplets of equal or smaller 5 µm suspended in a gas are called aerosol in contrast to larger particles typically above 10 µm (B. Bake, et. al., Respiratory research 20, 8-8 (2019)). In the following, referring to the exhaled breath, both, small particles and large particles can either be liquid droplets or dried-out droplet nuclei/residues after evaporation of the aqueous phase. As used herein, aerosol includes both small and large droplets. Large droplets with a size larger than 100 µm drop to the ground before they evaporate, causing local contamination. In case of disease transmission, the transmission through these large droplets is often referred to as droplet or contact spread. Namely, disease transmission occurs because you touch a surface contaminated by these droplets, or get caught within the spray zone when the patient is coughing. Small particles (small droplets) are so small that due to other forces, gravity is overcome, allowing them to stay suspended in the air for long periods or they evaporate before they hit the floor leaving the solid particulate which is also called the droplet nuclei free to flow very long distances, causing the so called airborne transmission. Respiratory droplets as part of the respiratory air are created by various mechanisms, e.g. vocal cord vibration within the larynx during speaking or reopening of collapsed terminal airways during breathing. Spreading by aerosols results in airborne transmission as infection route while typically microorganisms are spread through contact or droplets at approximately below 5 $\mu$m. It has been reported that as many as 80 - 90 % of droplets generated by human exhalation are smaller than 10 $\mu$m. These droplets are typically produced while speaking, coughing and sneezing which droplets are sufficiently small to remain airborne.

For example influenza RNA has been detected in aerosol particles from infected patients while just breathing comfortably. Further, influenza has been found in aerosols in random samples of air around emergency departments during flu season. That is, there are multiple lines of evidence that for influenza airborne transmission is possible. It is speculated that the same is true for corona virus transmissions, including SARS, SARS-2 and MERS. Moreover, what should be taken into consideration is, that small droplets present in the aerosol will be most concentrated within a few meters of the breathing or exhaling person. At that distance, small droplet spread (airborne spreading) is almost indistinguishable from large droplet spread (droplet spreading).

Although SARS and MERS spread primarily by large droplets, there were also outbreaks that were best explained by airborne spread of the disease. Also for Covid-19 airborne transmission is considered as being the source for infection.

Hence, determining and analysing components and solids present in respiratory droplets, in particular, microorganisms like bacteria and viruses is of help to identify infectious but asymptotic subjects, in particular, to avoid further spreading either airborne or droplet spreading of pathogens. This addresses several unmet needs for example as therapy companion in clinical treatments of airborne diseases differentiating true pathogen positive individuals from nucleic acid positives using a non-invasive sample technique. Additionally allowing a user-tolerated, safe and high-throughput as well as non-invase identification of asymptomatic carries of airborne pathogens in medical and on medical environments (e.g. airports, train stations and schools). Furthermore, the proposed technology can be used as well as in large epidemiological field studies identifying the burden of airborne infectious diseases.

To allow the detection of components and solids, in particular, microorganisms in aerosol with droplets from the respiratory air of a subject, various non-invasive based apparatus, systems and/or methods have been developed. Typically, filter material is used to enrich these microorganisms. For example, WO 2010/075265 A2 describes a lung aerosol collection device with a complex apparatus finally capturing the aerosol by filter. Other devices for aerosol collection and respective methods are described in WO 2012/068374 A2 also based on a membrane filter. A portable sampling device and method for detection of biomarkers in exhaled breath is described in WO 2013/132085 A1 wherein a sampling membrane is ranged in a filter unit allowing a collection of the aerosol from the exhaled breath.

Moreover, other methods and devices are based on collecting an condensate from the exhaled air, the so called exhaled breath condensate (EBC), whereby exhaled water vapour that is condensed by means of low temperature sampling namely cooled temperature below ambient temperature requiring additional equipment, both volatile and non-volatile components and particles. However, EBC is connected with a number of methodological difficulties and, thus, is not the method of choice.

Hence, there is an ongoing need for suitable methods and assemblies allowing sampling of droplets and aerosols from respiratory air whereby in the droplets and aerosols microorganisms, in particular, pathogens, are present.

## Brief description of the present invention

In a first aspect, an assembly for enrichment of microorganisms is provided, said assembly allows enrichment of microorganisms present in aerosol with droplets from the respiratory air of a subject, with at least one filter unit, at least one inlet and at least one outlet, at least one of the filter units comprising a first region containing beads, wherein droplets present in the aerosol attach to the beads when respiratory air is introduced by the inlet and passed into the filter unit through the inlet on exhalation.

In a further aspect, a method for enrichment of microorganisms from respiratory air of a subject comprising the steps of:

  a. Providing an assembly having at least one filter unit, at least one inlet and at least one outlet;
  b. Collecting aerosol with droplets containing microorganisms from the respiratory air in a filter unit upon exhalation of the subject by passing the exhaled air through at least one inlet to the at least one filter unit, the filter unit having a region with beads for enriching the microorganisms present in the aerosol including droplets.

Moreover, a method for determining microorganisms, in particular, pathogens, in the respiratory air of a subject is described comprising the steps of collecting the microorganisms, in particular, the pathogens from the aerosol including small droplets from the exhaled air of a subject within an assembly with at least one filter unit, at least one inlet and at least one outlet, the filter region comprising a region containing beads, in particular, with the assembly according to the present invention, and, separating the microorganisms attached on the beads and determining the microorganisms, in particular, the pathogens by appropriate methods including microbiological and molecular biological methods.

Further, the use of an assembly according to the present invention for the enrichment of microorganisms present in the aerosol or including small droplets from the respiratory air of the subject and for determining the presence of microorganisms in the respiratory air of a subject is described.

**Brief description of the drawings**

Figure 1 shows the conceptional build and functionality of the filter unit including the direction of the airflow and showing particles within the airflow.

Figure 2 shows the Assembly containing at least one filter unit, at least one inlet and at least one outlet.

Figure 3 displays the performance improvement auf the bead-based filter cartridge design in comparison to a standard planar PVDF-filter membrane (0.2 $\mu$m pore size, WHATMAN).

Figure 4. Performance of filter cartridge regarding relation-ship between pressure drop and airflow rate (A) and between deposition efficiency and particle size at an airflow rate of 30 l/min (B).

**Detailed description of the present invention**

In a first aspect, the present invention relates to an assembly for enrichment of microorganisms present in the aerosol with small droplets from the respiratory air of a subject, with at least one filter unit, at least one inlet and at least one outlet, at least one of the filter units comprising a first region containing beads, wherein the aerosol including small droplets attach to the beads when respiratory air is introduced by the inlet and passed into the filter unit through the inlet on exhalation.

As used herein, the term "assembly" is used interchangeably with the term "arrangement" unless otherwise indicated. Namely, the assembly is composed of several components or parts. The assembly contains or comprises at least one filter unit with at least one inlet and at least one outlet. The assembly may contain additional components or may contain more than one of the components mentioned above, namely, the filter unit, the inlet or outlet. The assembly may be one piece, e.g. for single use or may be multipart. In particular, the filter unit may be a removable filter unit, like a removable filter cartridge.

Namely, the assembly does not require any condensation of liquid in the exhaled air of a subject. In contrast, safe and easy enrichment directly from the aerosol present in the respiratory air after exhalation is possible.

As used herein, the term "contain" or "containing" as well as the term "comprise" or "comprising" which are used interchangeably, include the embodiments "consists of" or "consisting of".

Further, the term "determining" as used herein refers to accessing the presence, absence, quantity, level or amount of the components or particles in question, in particular, the microorganisms.

The term "droplets" or "droplet" as used herein refers to droplets generated in the respiratory tract and which can be either small in the size range below 5 $\mu$m (small droplets) or larger with sizes above 5 $\mu$m (large droplets).

The term "aerosol" as used herein refers to: exhaled air containing endogenously generated droplets of different sizes including small droplets and large droplets as well as droplet-nuclei suspended in a gas.

Moreover, the term "size" or "particle size" refers to the size of the respective droplets or droplet nuclei or particles determined by known means or defined by known means representing the mean size. The same is true for the particle size of the beads present in the filter unit wherein the size identifies that mean particle size as determined by known means.

Further, the phrase "aerosol including droplets attached to the beads" refers to the adherence of the nongaseous components of the aerosol including the small and large droplets as well as the droplet nuclei generally referred to particles.

The term "particle" or "particles" as used herein refers to the non-gaseous components present in the aerosol including the small and large droplets as defined herein as well as droplet-nuclei, said particles adhere on the surface of the beads or in case of porous beads adherence to the surface or at least being partly present in the pores.

As used herein, the term "microorganisms" include bacteria, viruses, fungi.

The term "beads" refers to a material which is in an embodiment basically spherical formed. However, all forms of beads are possible. The beads may be of different size, and different types of beads (either being different in material and/or being different in size) are encompassed.

That is, the present inventors recognized that with the use of beads present in filter units it is possible to separate components or particles present in the aerosol in particular present in the droplets from the respiratory air on exhalation. While the respiratory particles (small or large droplets and droplet nuclei) with the microorganism present in aerosol, droplets or droplet nuclei adhere on the beads, the gaseous components pass the filter and leave via the outlet. The particles are enriched whereby the particles include microorganisms. Thus, it is possible to enrich the microorganisms and separate these microorganisms from the exhaled air while being present in the small and large droplets or droplet nuclei.

By enrichment of the microorganisms which were present as solids in the aerosol like present in the droplets, it is possible to characterize and analyse these microorganisms further. Firstly, since the respiratory droplets allow to keep the microorganisms in a moist environment, in particular upon closing/sealing the filter cartridge, it is possible to analyse living microorganisms. Secondly, the presence in the liquid of the droplets allows to promote adherence to the beads while gaseous fractions pass the filter unit. The majority of non-volatile droplet compounds will stay in close proximity or attached to the beads. These compounds include biological functional or partially degraded molecules such as derivates of proteins, nucleic acids, lipids, sugars, electrolytes and lipids.

The filter unit may comprise different regions whereby in a first region the beads are present. In another region of said filter unit, a material for stabilizing the beads may be present. The material for stabilizing the beads may be any suitable material allowing passing the respiratory air, the aerosol, with droplets or droplet nuclei while providing other mechanical properties to the filter system and/or retaining the beads in the filter system. For example, the material allows to improve the mechanical resistance but also influence the pressure of the exhaled air. Suitable material includes: polymers such as poylstyrene or polypropylene, metals such as copper or gold.

In an embodiment, the filter unit contains a removable filter cartridge with respective regions containing the beads, and if present, the material for stabilizing the beads and/or retaining the beads in the filter system. Namely, in an embodiment, the assembly is an assembly wherein the filter unit contains a removable filter cartridge with at least one region containing the beads and, optionally, at least a further region containing material for stabilizing or providing other properties to the filter cartridge.

Moreover, the filter unit (for example the removable filter cartridge) comprises a second region containing beads wherein the beads of the first region and the second region may differ in physical or chemical properties. For example, the beads present in the first region and the at least second region differ in particle size or chemical properties for selective enrichment of microorganisms. For example, based on these different properties it is possible to enrich different types of microorganisms, like different types of viruses in different regions of the filter unit. For example, the beads may be of different material resulting in different adherence of the aerosol or droplets and droplet nuclei, respectively. Further, chemical modifications of the beads allow to improve deposition of the droplets or droplet nuclei and their constituents, in particular, the microorganisms and pathogens, present in the aerosol accordingly.

In an embodiment of the present invention, the filter unit allows to enrich microorganisms present in the small aerosol particle (small droplets), e.g. present in the lower airways while larger liquid droplets or droplet nuclei are separated in the second region filled with beads different in size and/or shape, e.g. present in the upper airways.

That is, in an embodiment of the present invention, the assembly and the method of the present invention allow to adhere small droplets while larger droplets or droplet nuclei pass the filter unit.

The beads according to the present invention are in an embodiment silica beads including glass beads. Of course, other materials like beads based on polymers can be used, e.g., polysterene.

In addition, the beads, like the silica beads, are non-functionalized or at least partly functionalized, e. g., in one region of the regions containing beads present in the filter, the beads, like the silica beads, are partly functionalized while other beads present in a different region and/or mixed with the functionalized beads above are not functionalized.

In an embodiment of the present invention, beads being functionalized are beads functionalized by silanes. Examples of silanes useful for functionalization of the beads, in particular, the silica beads, include Hexadecyltrimethoxysilane, Hexadecyltrietrhoxysilane, 3-Aminopropyltrimethoxysilane, 3-Aminopropyltriethoxysilane, Nonafluorohexyltrimethoxysilane, or Nonafluorohexyltriethoxysilane. Other silanes known for functionalization of silica based materials like silica beads can be used. The skilled person is well aware of suitable materials like silica beads can be used. The skilled person is well aware of suitable materials such as silanes containing head groups with strong and/or specific interactions with the target analyte (for example charges or polar head groups or antibody containing head groups or interaction mediated by protein-protein, protein-sugar moieties, protein-nucleic acid like lectin-binding, or Cas9-binding.

Either mixtures of different functionalized beads may be present in the same region of the filter unit or, alternatively, the functionalized or non-functionalized beads are present in different regions of the filter unit. In addition, it is possible to vary particle size and shape besides the functionalization of the beads.

In an embodiment, the bead particle size is in the range of from 0.1 to 20 mm , like 0.2 to 10 mm, e. g. 0.3 to 5.0 mm, and 0.5 to 2 mm. That is, depending on the microorganism to be enriched from the respiratory air or the region or respiratory manoeuvre to be sampled, the bead size may be selected. Of course, also the functionalization and the material may be selected based on the microorganism to be enriched.

For example, in case of gram positive and gram-negative bacterial species as well as eukaryotic pathogens like fungi and parasites, the particle size may be selected from 0.5 mm to 1 mm. A suitable range of particle sizes spans 0.1 mm to 1 mm in case for viruses.

In addition, based on the desired pressure drop and the air flow rates through the filter unit, the particle size and the functionalization and material of the beads is selected. Of course, stabilizing material, if present, can influence pressure drop and air flow rates accordingly.

In an embodiment of the present invention, the beads present in the assembly are composed of two fractions of different particle size, in an embodiment, the ratio of a first population of beads and a second population of beads having different particle sizes is in the range of from 80:20 to 20:80, like 70:30, 30:70, e. g., 60:40 to 40:60. Typically the difference between the particle sizes is at least 25 %, like 50 %, in particular, 100 %, e. g. 200 %. For example, the first population has a particle size of from 0.3 to 0.8 mm, while the second population has a particle size of 0.7 to 1.2 mm whereby both populations do not have the same size.

In another embodiment, the filter unit may contain more than one removable filter cartridges whereby each of the filter cartridges may contain a specific type of beads. That is, the assembly according to the present invention may contain two or more filter cartridges wherein in a first filter cartridge a first type of beads allows to enrich a first type of microorganisms whereas a second or further filter cartridge contains a different type of beads with different properties, e. g., with respect to size, functionalization or material, thus, allowing to enrich a different type of microorganism.

In an embodiment of the present invention, the assembly according of the present invention enables the enrichment of pathogenic microorganisms, like viruses, present in the particles of aerosol of exhaled respiratory air of a subject wherein the filter unit or the removable filter cartridge comprising a first region containing a mixture of beads of different particle sizes and shapes and, optionally, a second region containing beads of different particles sizes and shapes. This may allow to separate particles from different compartment of the airways, like the upper and lower airway. In case of a removable filter cartridge containing beads whereby microorganisms are enriched on said beads, the cartridge may be removed and may be processed further to analyse the microorganisms enriched in the filter unit. For example, the cartridge can be removed easily and processed by a self-closing housing of the filter cartridge. The housing or container allows to analyse the microorganisms, like pathogenic microorganisms, at a different place and can be transported safely. Therefore the cartridge is manufactured in a way to allow safe handling without direct contact to the contaminated surface within the filter.

Moreover, the enrichment by adherence of the microorganisms to the beads allow further extraction of the microorganisms for later analysis of the same. Hence, the assembly enables a direct pathogen detection from expirate or respiratory air.

In particular, it is possible with the assembly according to the present invention to detect respiratory microorganisms like respiratory pathogens also from the lower airways and lung, for example, collecting the respiratory samples from coughs / forced exhalation or normal breathing. Additionally, the collection of sputum as part of the coughing process can be collected as well.

Due to the adherence to the beads, the microorganisms can be recovered from the filter material for later analysis. This analysis may be any kind of known type of analysis including microbiological or molecular biological methods.

In a further aspect, the present invention relates to a method of enrichment of microorganisms from respiratory air of a subject comprising the step of

a) Providing an assembly having at least one filter unit, at least one inlet and at least one outlet;
b) Collecting particles present in the aerosol containing microorganisms from the exhaled respiratory air in a filter unit upon exhalation of the subject by passing the exhaled air through at least one inlet to the at least one filter unit, the filter unit having a region with beads for enriching the microorganisms present in the aerosol with the particles.

That is, the present invention refers preferably to a method for enrichment of microorganisms using the assembly according to the present invention.

The method for enrichment of microorganisms according to the present invention allows to enrich said microorganisms, in particular, pathogens like bacteria and viruses. The method based on the assembly according to the present invention enables easy handling and is user friendly. In addition, the method is a non-invasive method, thus, safe and easy handling is possible and, additionally, the practitioner is not exposed to possible pathogenic material.

The present invention relates in another aspect to a method for determining microorganisms, in particular, pathogens, in the respiratory air of a subject after exhalation comprising the steps of

a) Collecting the microorganisms, in particular, the pathogens from particles from the aerosol of the exhaled air of a subject within an assembly with at least one filter unit, at least inlet and at least one outlet, the filter region comprising a region containing beads allowing adherence of microorganisms, and

b) Separating the microorganisms from the beads determining the microorganisms, in particular, the pathogens by appropriate methods including microbiological and molecular biological methods.

In the method for determining microorganisms according to the present invention, the collection of microorganisms is conducted with the assembly according to the present invention. The separation of the microorganisms from the beads, namely, the recovery of the microorganisms, like the pathogens, from the filter, e. g., from the filter cartridge, can be conducted at a different place allowing handling of the pathogens and conducting the method for analysing the microorganisms accordingly. For example, detection of the pathogen may be by molecular biology including amplification methods, like PCR. Also cell culture is possible, thus, determining the CFU of the isolated microorganisms.

In an embodiment the method for determining microorganisms according to the present invention, in particular, for determining the presence of pathogens, like viruses, in the respiratory airways of a subject, comprises the step of collecting the pathogens, like a virus, with the filter unit, like a removable filter cartridge, as defined herein as a part of the assembly and separating the pathogens, in particular, the virus from the beads and determining the presence of the pathogens, in particular, viruses. For example in case of influenza but also with respect to corona virus, this method allows a safe and easy determination of the microorganism. Moreover, the method and assembly is user friendly, being non-invasive and does not require collection of fluid, like sputum, but enables determination of pathogens from the lower airway and lung aerosols. This is particularly true for determining and collecting pathogens from lower airway aerosols including smaller and larger droplets by forced exhalation or coughing from patients with low or without any sputum expectoration or from the lung during normal breathing.

Further, the present invention relates to the use of an assembly according to the present invention for the enrichment of microorganisms present in aerosol including smaller and larger droplets or droplet nuclei from the respiratory air of a subject. The use is particularly useful for the enrichment of microorganisms present in aerosol from the respiratory air of a subject.

Finally, the present invention relates to the use of an assembly according to the present invention for determining the presence of microorganisms in the respiratory air of a subject, in particular, for determining the presence of viruses like enveloped viruses. The assembly is particularly useful for enrichment and analysis of the presence of pathogens, like viruses. Namely, it is possible to enrich and analyse viruses, like corona virus including SARS, SARS_CoV-2 and MERS as well as influenza virus and other respiratory viruses suitable for airborne transmission.

The invention will be described further by reference to the figures.

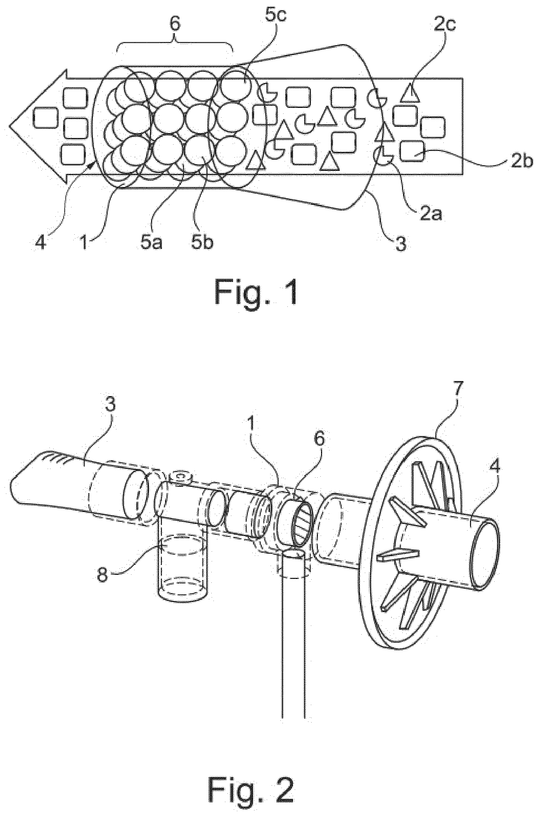

In figure 1 an assembly according to the present invention is shown. The assembly comprises a filter unit 1 with an inlet 3 and an outlet 4. The direction of airflow is shown by an arrow. Different types of particles 2a, 2b, 2c are shown. These particles pass the filter unit 1 and attach to the beads 5a, 5b, 5c present in the filter unit 1, while the gas leaves the filter unit 1 through the outlet 4.

Figure 2 shows another embodiment of the assembly according to the present invention with a filter cartridge 6 containing the filter unit (not shown), in the filter unit the beads are present. The direction of the airflow is shown by the arrow whereby the airflow with the aerosols is introduced into the assembly by inlet 3. The inlet 3 is coupled to the filter cartridge 6 present in the filter unit 1 by a coupling 8. The outlet 4 comprises a further filter unit 7.

Possible particles including larger and smaller droplets and droplet-nuclei attach to the beads present in the filter cartridge while the gaseous components of the aerosol pass through the filter unit leaving the assembly through the outlet passing the further filter unit 7.

The present invention will be described further by way of example without limiting the same.

Example 1

The performance of the bead-based filter cartridge was evaluated in comparison to a a planar filter membrane (PVDF) by aerosolizing 300 $\mu$l of a bacterial suspension of *Escherichia coli* K12 with a concentration of $1\times10^5$ cfu/ml. The volume was aerosolized in a nebulizer unit for 1 minute using a bead cartridge comprising a mix of beads with 0.5 mm (25%) and 1 mm diameter (75%) of total weight of 2.376 g. The Nebulizer was de-assembled and immobilized material attached to the beads was washed with 1 ml sterile PBS, incubate for 1 min in the closed 50 ml conical tube, centrifuge 30 min 300xg at RT in the swing out rotor with the brake switched on. The flow through was collected. This cycle was repeated without the addition of extra PBS after transfer the filtrate into a new Eppendorf tube. The number of viable bacteria after aerosolization, binding and washing was determined by plating and calculating the cfu/ml in the combined flow through fraction of the washing steps. The different cfu/ml of the bacterial suspension bevor and after aerosolization were used to calculate the loss factor according to equation 1.

Equation 1:

$$Loss = \frac{Input\ (\frac{cfu}{ml})}{Recovered\ (\frac{cfu}{ml})}$$

Loss .. Performance of the filter cartridge, whereas 1 is no loss and optimal performance and values >1 are loss due to non-bind. ing or non-detachment during the washing.

Input .. concentration of bacterial suspension before aerosolization

Recovered .. concentration of bacterial suspension of the combined flow through fractions

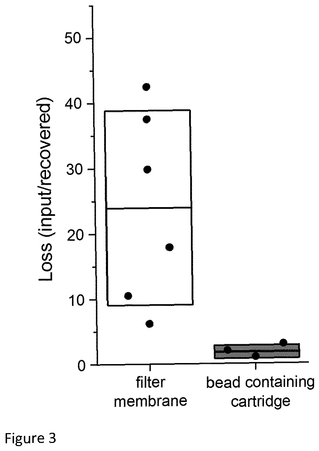

Result: The loss of the filter cartridge was 2 compared with a loss of 24.02 for the planar PVDF-filter membrane as shown in figure 3.

Example 2

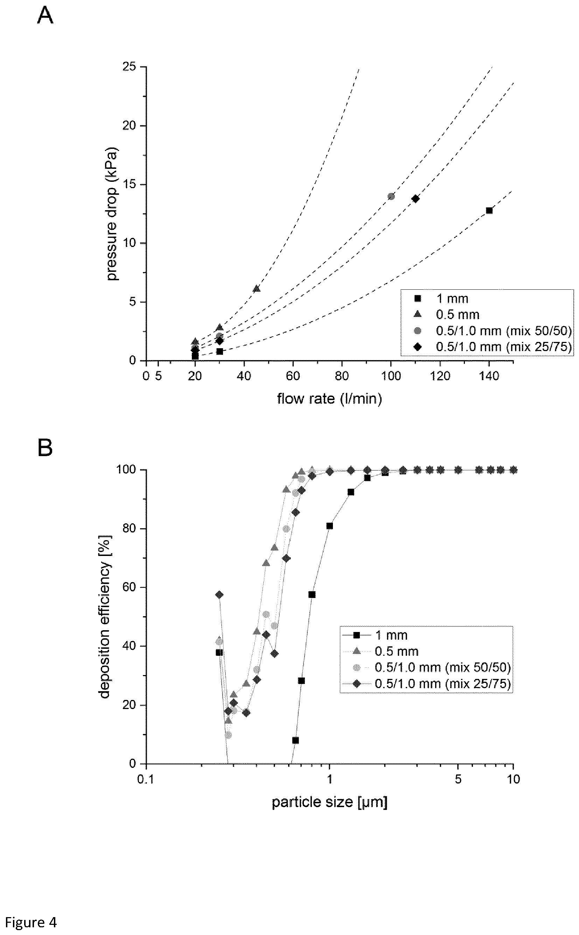

For the characterization of the pressure loss/resistance as well as the particle size-dependent deposition fraction has an experimental set-up been established. Using this set-up an optimization study was conducted considering the relevant breathing manoeuvres/flow rates, the particle-size dependent droplet deposition in the filter beads, the different bead sizes and mixtures thereof, filter cartridge design and the corresponding pressure losses (as shown in Fig. 4). Most important, with increasing flow rate and decreasing bead size both, deposition efficiency and pressure loss, increase significantly for the same volume of beads. According to these investigations, a cartridge with a diameter of about 17 mm and a length of about 7 mm, filled with a mixture of 25 % 0.5 mm and 75 % 1 mm beads results in a good performance (as shown in Fig. 5) and is assumed to be suitable for analysis of normal deep breathing and for coughing on the level of the peripheral lung as well as larger airways.

Using this configuration, an estimation with exhaled breath particles shows a mass-based droplet deposition efficiency of about 77 % at an airflow rate of 30 l/min.

List of reference signs

| 1 - | Filter unit |
| 2a, 2b, 2c ... - | Particles in the aerosol |
| 3 - | Inlet |
| 4 - | Outlet |
| 5a, 5b, 5c ... - | Beads |
| 6 - | Filter cartridge |
| 7 - | Filter |
| 8 - | Coupling |

**Claims**

1. Assembly for enrichment of microorganisms present in the aerosol in particular, in particles from the respiratory air of a subject, with at least one filter unit, at least one inlet and at least one outlet, at least one of the filter units comprising a first region containing beads, wherein the particles attach to the beads when respiratory air is introduced by the inlet and passed into the filter unit through the inlet on exhalation.

2. The assembly according to claim 1 wherein the filter unit contains further at least one region containing a material for stabilizing the beads.

3. The assembly according to claim 1 or 2 wherein the filter unit contains a removable filter cartridge with at least one region containing beads and, optionally at least a further region containing material for stabilizing or providing other properties to the filter cartridge.

4. The assembly according to any one of the preceding claims wherein the filter unit or the filter cartridge comprises a second region containing beads wherein the beads of the first region and the second region differ in particle size or chemical properties for selective enrichment of microorganisms.

5. The assembly according to any one of the preceding claims wherein the beads are silica beads.

6. The assembly according to any one of the preceding claims wherein the silica beads are at least partly functionalized, like in one region of the regions containing beads present in the filter unit or the filter cartridge functionalized silica beads.

7. The assembly according to claim 6 wherein the beads are functionalized by silanes.

8. The assembly according to any one of the preceding claims wherein the bead particle size is in the range of from 0.1 mm to 20 mm, like 0.2 mm to 10 mm, e.g. 0.3 mm to 1 mm.

9. The assembly according to any one of the preceding claims wherein the beads are composed of two fractions of different particle size, preferably, wherein the ratio of a first population of beads and a second population of beads having different particle sizes is in the range from 80:20 to 20:80.

10. The assembly according to any one of the preceding claims for the enrichment of pathogenic microorganisms, like viruses, present in the aerosol, in particular in the small droplets, from the respiratory air of a subject wherein the filter unit or the removable filter cartridge comprising a first region containing a mixture of beads of different particle size and, optionally, a second region containing beads.

11. A method for enrichment of microorganisms from respiratory air of a subject comprising the steps of:

   a. Providing an assembly having at least one filter unit, at least one inlet and at least one outlet;
   b. Collecting particles present in the aerosol containing microorganisms from the respiratory air in a filter unit upon exhalation of the subject by passing the exhaled air through at least one inlet to the at least one filter unit, the filter unit having a region with beads for enriching the microorganisms present in the aerosol with the particles.

12. The method according to claim 11 wherein the assembly as an assembly according to any one of claims 1 to 10.

13. The method according to claim 11 or 12 wherein the microorganisms are pathogens, in particular, bacteria, viruses and fungi.

14. A method for determining microorganisms, in particular, pathogens, in the respiratory air of a subject comprising the steps of
collecting the microorganisms, in particular, the pathogens from the particles from the aerosol of the exhaled air of a subject with an assembly with at least one filter unit, at least one inlet and at least one outlet, the filter region comprising a region containing beads, in particular, with the assembly according to any one of the claims 1 to 10, separating the microorganisms from the beads and determining the microorganisms, in particular, the pathogens by appropriate methods including microbiological and molecular biological methods.

15. The method according to claim 14 for determining the presence of pathogens, in particular, viruses in the respiratory air of a subject comprising the steps of collecting the pathogens, in particular, the virus with a filter unit, like a removable filter cartridge according to any one of claims 1 to 10;
separating the pathogens, in particular, the virus, from the beads and determining the presence of pathogens, in particular, viruses.

16. The use of an assembly according to any one of the claims 1 to 10 for i) the enrichment of microorganisms present in particles of the aerosol from the respiratory air of a subject and/or ii) for determining the presence of microorganisms in the respiratory air of a subject, in particular, for determining the presence of viruses like enveloped viruses.

Fig. 1

Fig. 2

Figure 3

A

B

Figure 4

Europäisches
Patentamt

European
Patent Office

Office européen
des brevets

**EUROPEAN SEARCH REPORT**

Application Number

EP 20 21 3820

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2012/006250 A1 (DETON CORP [US]; SISLIAN PATRICK [US] ET AL.) 12 January 2012 (2012-01-12) * claims 1-26; figures 1, 3, 6 * | 1-16 | INV. C12Q1/24 A61B10/00 G01N33/497 G01N33/569 |
| X | US 2015/184224 A1 (GUY MICHEL [FR] ET AL) 2 July 2015 (2015-07-02) | 1-3,5,6, 8,10-16 | |
| Y | * paragraphs [0058], [0144], [0145], [0182]; claims 1-16; figures 2, 5-9 * | 1-16 | |
| X | US 2017/119280 A1 (AHMAD LUBNA M [US] ET AL) 4 May 2017 (2017-05-04) * paragraphs [0033] - [0099]; figures 16, 27, 28, 51 * | 1,2,5,6 | |
| Y | SANDE MARIA G ET AL: "New solutions to capture and enrich bacteria from complex samples", MEDICAL MICROBIOLOGY AND IMMUNOLOGY, SPRINGER, DE, vol. 209, no. 3, 5 February 2020 (2020-02-05), pages 335-341, XP037147027, ISSN: 0300-8584, DOI: 10.1007/S00430-020-00659-1 [retrieved on 2020-02-05] * the whole document * | 1-16 | |

TECHNICAL FIELDS SEARCHED (IPC)

G01N
C12Q
A61B

The present search report has been drawn up for all claims

| Place of search | Date of completion of the search | Examiner |
|---|---|---|
| Munich | 28 April 2021 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 21 3820

This annex lists the patent family members relating to the patent documents cited in the above-mentioned European search report.
The members are as contained in the European Patent Office EDP file on
The European Patent Office is in no way liable for these particulars which are merely given for the purpose of information.

28-04-2021

| Patent document cited in search report | | Publication date | Patent family member(s) | | Publication date |
|---|---|---|---|---|---|
| WO 2012006250 | A1 | 12-01-2012 | CN | 103119417 A | 22-05-2013 |
| | | | EP | 2591331 A1 | 15-05-2013 |
| | | | US | 2013217029 A1 | 22-08-2013 |
| | | | WO | 2012006250 A1 | 12-01-2012 |
| US 2015184224 | A1 | 02-07-2015 | CN | 104583752 A | 29-04-2015 |
| | | | EP | 2872867 A1 | 20-05-2015 |
| | | | FR | 2993281 A1 | 17-01-2014 |
| | | | JP | 5993516 B2 | 14-09-2016 |
| | | | JP | 2015523081 A | 13-08-2015 |
| | | | US | 2015184224 A1 | 02-07-2015 |
| | | | US | 2020190561 A1 | 18-06-2020 |
| | | | WO | 2014009673 A1 | 16-01-2014 |
| US 2017119280 | A1 | 04-05-2017 | US | 2017119279 A1 | 04-05-2017 |
| | | | US | 2017119280 A1 | 04-05-2017 |
| | | | US | 2017224251 A1 | 10-08-2017 |
| | | | US | 2019261891 A1 | 29-08-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010075265 A2
- WO 2012068374 A2
- WO 2013132085 A1

**Non-patent literature cited in the description**

- **B. BAKE.** *Respiratory research,* 2019, vol. 20, 8-8